Europäisches Patentamt

European Patent Office

Office européen des brevets

(11) Numéro de publication: **0 456 945 A1**

## (12) DEMANDE DE BREVET EUROPEEN

(21) Numéro de dépôt: 90470026.7

(22) Date de dépôt: 16.05.90

(51) Int. Cl.⁵: **A61M 5/32**

(43) Date de publication de la demande:
**21.11.91 Bulletin 91/47**

(84) Etats contractants désignés:
**AT BE CH DE DK ES FR GB GR IT LI LU NL SE**

(71) Demandeur: **Kinnel, Marc**
**2 rue de la Fontaine**
**F-57420 Coin sur Seille(FR)**

(72) Inventeur: **Kinnel, Marc**
**2 rue de la Fontaine**
**F-57420 Coin sur Seille(FR)**

(74) Mandataire: **Poupon, Michel**
**3, rue Thiers BP 421**
**F-88011 Epinal Cédex(FR)**

(54) Dispositif manuel individuel pour la manipulation et le retrait d'aiguilles d'injection ou de prélèvement usagées.

(57) Dispositif manuel individuel pour la manipulation et le retrait d'aiguilles d'injection ou de prélèvement usagées permettant d'éviter tout contact de celles-ci avec l'utilisateur, du type comprenant au moins un mors venant maintenir l'aiguille pendant son retrait, caractérisé en ce qu'il comporte essentiellement ;

- un manche (1) de préhension par l'utilisateur,
- un embout conique (3) de réception de l'aiguille comportant un mors fixe. (8)
- un mors mobile (6) commandé par l'utilisateur et venant verser l'aiguille (13) en coopérant avec le mors fixe. (8)

FIG. 1  FIG. 2

La présente invention a pour objet un dispositif manuel individuel pour la manipulation et le retrait d'aiguilles d'injection ou de prélèvement usagées permettant d'éviter tout contact de celles-ci avec l'utilisateur, du type comprenant au moins un mors venant maintenir l'aiguille pendant son retrait.

Le problème à résoudre consiste à éviter les risques de blessure par piqure ou par contact avec des aiguilles usagées, en particulier pour éviter la transmission de maladies telles que le SIDA ou autres maladies virales ou infectieuses.

A cette fin, il est indispensable de stocker les aiguilles usagées dans des récipients imperforables, afin d'éviter des accidents lors du transport des déchets.

La majorité des blessures survenant lors de la repose du capuchon de l'aiguille ou de la déconnexion de l'aiguille à la main, il s'avère donc indispensable d'éliminer tout contact entre les mains de l'usager et l'aiguille.

Il existe déjà des dispositifs permettant d'obtenir un tel résultat.

Ainsi on a décrit dans le brevet US 4 738 362 un système à récipients en matière plastique possédant un ou plusieurs orifices dans lesquels se fichent les aiguilles.

Après déconnexion de la seringue, l'aiguille tombe dans le container. Les containers sont détruits après remplissage.

Ce dispositif est très volumineux et de manipulation peu pratique.

Dans une seconde famille de dispositifs, décrits dans les demandes PCT WO 88/08313 et WO 88/06133 on propose des tubes oblongs dans lesquels on introduit l'aiguille à retirer, qui est extraite par maintien avec un mors escamotable solidaire dudit tube.

Ces dispositifs présentent l'inconvénient d'être de forme géométrique peu large.

Donc il existe un risque certain pour l'utilisateur, qui doit maintenir le corps du tube d'une main, de voir la seringue glisser sur la partie haute du tube et donc blesser la main qui tient celui-ci.

La présente invention a pour but de remédier à ces inconvénients et de proposer un dispositif qui soit entièrement fiable et de conception simple, de fonctionnement aisé et facilement compréhensible pour l'utilisateur.

Conformément à l'invention, ce résultat est obtenu avec un dispositif manuel individuel pour la manipulation et le retrait d'aiguilles d'injection ou de prélèvement usagées permettant d'éviter tout contact de celles-ci avec l'utilisateur, du type comprenant au moins un mors venant maintenir l'aiguille pendant son retrait, caractérisé en ce qu'il comporte essentiellement :

- un manche de préhension par l'utilisateur,
- un embout conique de réception de l'aiguille comportant un mors fixe,
- un mors mobile commandé par l'utilisateur et venant serrer l'aiguille en coopérant avec le mors fixe.

On comprendra mieux l'invention à l'aide de la description faite ci-après de deux modes de mise en oeuvre donnés à titre d'exemples non limitatifs, en référence aux dessins annexés dans lesquels :

- la figure 1 est une vue de dessus d'un dispositif conforme à l'invention selon un premier mode de mise en oeuvre, en coupe longitudinale selon A-A ;
- la figure 2 est une vue en élévation latérale en coupe du dispositif de la figure 1 ;
- la figure 3 est une vue arrière du dispositif de la figure 1 ;
- la figure 4 est une vue de dessus d'une variante de mise en oeuvre ;
- la figure 5 est une vue en élévation latérale d'une variante de mise en oeuvre du dispositif de l'invention ;
- la figure C est une vue de dessus du dispositif de la figure 5 en coupe partielle selon B-B.

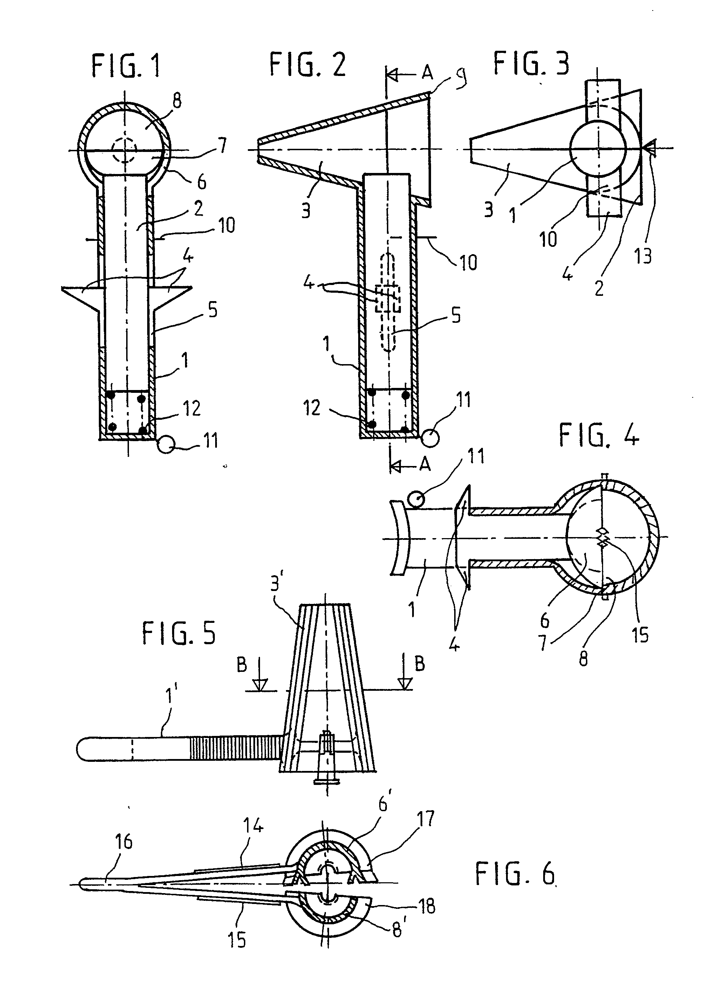

En référence aux figures 1, 2 et 3, le dispositif comporte un manche (1) prolongé à l'avant par un tronc de cône (3) placé perpendiculairement au manche (1).

Deux leviers mobiles (4) formant poignées situés de part et d'autre du manche (1) compriment un ressort (12) placé à l'arrière de l'intérieur du manche en entraînant une plaque mobile crantée (6), ou mors, solidaire d'un corps cylindrique coulissant (2) libérant un orifice (15) au centre de la grande base du tronc de cône (3) et permettant l'introduction de l'aiguille usagée (13) dans le tronc de cône. Cette plaque mobile (6) coulisse à l'intérieur d'une gorge (7) située sur la grande base du tronc de cône (3). Les leviers (4) coulissent dans des lumières (5) du manche (1).

Lorsque le ressort (12) est relaché, il repousse la plaque mobile (6) contre une autre plaque crantée fixe (8) ou mors immobilisant ainsi l'aiguille entre les deux mors.

Un carter de protection (9) situé sur la grande base du tronc de cône (3) chevauchant l'arrière de la plaque mobile (6) recouvre l'espace nécessaire au mouvement de celle-ci. Un carter (10) recouvre l'avant du manche (1) le séparant du tronc de cône (3).

Avantageusement le manche (1) comporte un moyen de suspension tel qu'un anneau (11).

Dans la variante du dispositif selon l'invention représentée à la figure 4 l'appui de l'index et du majeur sur les leviers (4) et du pouce à l'arrière du manche (1) crée une ouverture par retrait de la plaque crantée mobile (6).

Le ressort (12) est circulaire et entoure la plaque fixe en passant dans la gorge (7) et la plaque mobile (6) en passant à travers les leviers (4).

Le fonctionnement est le suivant.

Les deux leviers situés de part et d'autre du milieu du manche de l'appareil sont tirés vers l'arrière le long des lumières (5) prévues à cet effet dans le manche.

Lors de leur retrait vers l'arrière de l'appareil, ils ramènent le corps cylindrique (2) dont une extrémité support la plaque mobile (6) crantée en son centre, libérant ainsi un espace permettant l'introduction de l'extrémité effilée de l'aiguille au centre de la grande base du tronc de cône.

Le ressort (12) est alors comprimé.

En relachant les leviers, ledit ressort se détend et repousse vers l'avant le corps cylindrique au bout duquel est fixé la plaque mobile crantée en son centre.

L'aiguille est alors bloquée par cette plaque mobile contre une plaque identique mais fixée sur la grande base du tronc de cône.

L'aiguille se trouve ainsi dans le tronc de cône, celui-ci la recouvrant entièrement, il suffit alors de désolidariser l'aiguille de son support.

Un carter de protection recouvre le tiers avant de la grande base du tronc de cône supprimant ainsi l'ouverture nécessaire au mouvement de ladite plaque vers l'arrière. La forme conique du réceptacle à aiguille permet de recentrer cette dernière.

En présentant la petite base du tronc du cône contenant l'aiguille au-dessus d'un récipient de récupération et, en actionnant à nouveau les leviers vers l'arrière, l'aiguille libérée, tombe d'elle-même dans le récipient.

Ce dispositif peut être réalisé en métal inoxydable ou en plastique et de ce fait peut être, selon les cas, stérilisé à chaud ou à froid.

Dans la variante des figures 5 et 6, on retrouve le manche (1'), le cône (3') et deux mors (6', 8').

Dans cette variante, le rappel élastique est assuré par le manche (1') en deux parties (14,15) articulées en leur extrémité (16).

Le fonctionnement est donc identique au précédent, le cône étant réalisé en deux pièces qui s'emboitent (17,18).

**Revendications**

1. Dispositif manuel individuel pour la manipulation et le retrait d'aiguilles d'injection ou de prélèvement usagées permettant d'éviter tout contact de celles-ci avec l'utilisateur, du type comprenant au moins un mors venant maintenir l'aiguille pendant son retrait, caractérisé en ce qu'il comporte essentiellement ;
    - un manche (1) de préhension par l'utilisateur,
    - un embout conique (3) de réception de l'aiguille comportant un mors fixe,
    - un mors mobile commandé par l'utilisateur et venant verser l'aiguille en coopérant avec le mors fixe.

2. Dispositif selon la revendication 1, caractérisé en ce qu'il comporte deux leviers mobiles (4) formant poignées situés de part et d'autre du manche (1) comprimant un ressort (12) placé à l'arrière de l'intérieur du manche en entrainant une plaque mobile crantée (6), solidaire d'un corps cylindrique coulissant (2) libérant un orifice (15) au centre de la grande base du tronc de cône (3) et permettant l'introduction de l'aiguille usagée (13) dans le tronc de cône, ladite plaque mobile (6) coulissant à l'intérieur d'une gorge (7) située sur la grande base du tronc de cône (3), les leviers (4) coulissant dans des lumières (5) du manche (1).

3. Dispositif selon la revendication 2, caractérisé en ce que lorsque le ressort (12) est relaché, il repousse la plaque mobile (6) contre une autre plaque crantée fixe (8) immobilisant ainsi l'aiguille entre les deux mors.

4. Dispositif selon la revendication 1, caractérisé en ce que le cône (3') est réalisé en deux pièces (17,18) comportant chacune un mors (6',8'), le rappel élastique étant assuré par le manche (1') en deux parties (14,15) articulées en leur extrémité (16).

FIG. 1

FIG. 2

FIG. 3

FIG. 4

FIG. 5

FIG. 6

4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| E | FR-A-2 639 235  (M. KINNEL)<br>* En entier * | 1-3 | A 61 M 5/32 |
| | — — — | | |
| X,D | WO-A-8 808 313  (McCAMMON et al.)<br>* Figures 5,6; page 6, ligne 9; page 7, lignes 6-18 * | 1 | |
| Y,D | | 4 | |
| | — — — | | |
| Y | EP-A-0 191 113  (VEGA GRIESHABER GmbH & CO.)<br>* Figures 5-7; page 14, lignes 6-19 * | 4 | |
| | — — — | | |
| A,D | US-A-4 738 362  (BURNS et al.)<br>* Figures 1-6; colonne 4, lignes 45-54; colonne 5, lignes 3-13 * | 2,3 | |
| | — — — — — | | |

|  | DOMAINES TECHNIQUES RECHERCHES (Int. Cl.5) |
|---|---|
|  | A 61 M |

Le présent rapport de recherche a été établi pour toutes les revendications

| Lieu de la recherche | Date d'achèvement de la recherche | Examinateur |
|---|---|---|
| La Haye | 10 janvier 91 | SEDY, R. |